(19)

**Europäisches Patentamt**
**European Patent Office**
**Office européen des brevets**

(11) **EP 4 769 421 A1**

(12) **EUROPEAN PATENT APPLICATION**
published in accordance with Art. 153(4) EPC

(43) Date of publication:
**01.07.2026  Bulletin 2026/27**

(21) Application number: **24857877.5**

(22) Date of filing: **27.05.2024**

(51) International Patent Classification (IPC):
**G16H 20/17** *(2018.01)*

(52) Cooperative Patent Classification (CPC):
**G16H 20/17**

(86) International application number:
**PCT/CN2024/095389**

(87) International publication number:
**WO 2025/044331 (06.03.2025 Gazette 2025/10)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority:  **25.08.2023  PCT/CN2023/114922**

(71) Applicant: **Medtrum Technologies Inc.
Shanghai 201203 (CN)**

(72) Inventor: **YANG, Cuijun
Shanghai 201203 (CN)**

(74) Representative: **Vogelbruch, Keang
VOGELBRUCH Patentanwaltsgesellschaft mbH
Paul-Gerhardt-Straße 16
40593 Düsseldorf (DE)**

(54) **CLOSED-LOOP ARTIFICIAL PANCREAS SYSTEM FOR SAFE INFUSION**

(57)    The invention discloses a closed-loop artificial pancreas system for safe infusion, during a long period of real-time blood glucose value loss, the closed-loop artificial pancreas system can enter a learning mode or a safe mode. Based on historical blood glucose values and finger blood glucose values, it continues to calculate the required insulin infusion amount for the patient and infuses an appropriate amount of insulin to the patient. It will not infuse an inappropriate amount of insulin or even interrupt insulin infusion due to the lack of real-time blood glucose value, avoiding life-threatening situations for the patient and benefiting the patient's treatment.

**FIG.10**

**Description**

## TECHNICAL FIELD

The invention mainly relates to the field of diabetes monitoring and management, and in particular to a closed-loop artificial pancreas system for safe infusion.

## BACKGROUND

The pancreas of healthy people can automatically secrete the required insulin/glucagon according to the glucose level in the human blood, thereby maintaining a reasonable range of blood glucose fluctuations. However, for diabetic patients, the function of their pancreas has been severely compromised, and the pancreas cannot secrete the required dosage of insulin. Therefore, diabetes mellitus is defined as a metabolic disease caused by abnormal pancreatic function, and it is also classified as one of the top three chronic conditions by the WHO. The present medical advancement has not been able to find a cure for diabetes mellitus. Yet, the best the technology could do is control the onset symptoms and complications by stabilizing the blood glucose value for diabetes patients.

Diabetic patients on an insulin pump need to check their blood glucose before infusing insulin into their bodies. At present, most detection methods of continuously monitoring blood glucose are based on in vivo glucose monitoring devices, which use disposable transdermal sensors inserted into the skin to measure glucose concentrations in interstitial fluids and send the blood glucose value through a transmitter to the remote device in real-time for the user to view. This monitoring method is called Continuous Glucose Monitoring (CGM).

Closed-loop artificial pancreas system is a better choice for adjuvant treatment of diabetes patients. The closed-loop artificial pancreas system can not only conduct real-time blood glucose monitoring, but also automatically calculate and infuse insulin based on real-time blood glucose monitoring data, providing great convenience for patients with diabetes treatment. However, in the actual use process, the closed-loop artificial pancreas system will be unable to receive real-time blood glucose monitoring signals for a period of time due to unstable signals, sensor damage and other reasons. In this use scenario, the closed-loop artificial pancreas system in the existing technology often uses the way of reducing the fixed proportion of insulin infusion and suspending insulin infusion to spend this period of time, which cannot meet the patient's diabetes treatment, and may even put the patient in danger of life.

Therefore, there is an urgent need for a closed-loop artificial pancreas system that can safely administer insulin during periods of real-time blood glucose monitoring signal loss in existing technology.

## BRIEF SUMMARY OF THE INVENTION

The invention discloses a closed-loop artificial pancreas system for safe infusion, during a long period of real-time blood glucose value loss, the closed-loop artificial pancreas system can enter a learning mode or a safe mode. Based on historical blood glucose values and finger blood glucose values, it continues to calculate the required insulin infusion amount for the patient and infuses an appropriate amount of insulin to the patient. It will not infuse an inappropriate amount of insulin or even interrupt insulin infusion due to the lack of real-time blood glucose value, avoiding life-threatening situations for the patient and benefiting the patient's treatment.

The present embodiment discloses a closed-loop artificial pancreas system, comprising a detection mechanism, used to monitor real-time blood glucose value. A program mechanism, used to receive real-time blood glucose values from the detection mechanism and calculate insulin infusion volume based on the real-time blood glucose. And an infusion mechanism, used to implement insulin infusion. Wherein, when the program mechanism does not receive the real-time blood glucose value for the first period of time, the closed-loop artificial pancreas system switches from the first mode to the second mode for the second period of time. In the second mode, the closed-loop artificial pancreas system calculates the estimated blood glucose value, calculates the estimated insulin infusion volume based on the estimated blood glucose value, and completes the insulin infusion based on the estimated insulin infusion volume.

According to one aspect of the invention, the estimated blood glucose value is calculated based on real-time blood glucose values from the last period of time.

According to one aspect of the invention, it further comprising providing finger blood glucose values, the estimated blood glucose values being calculated based on finger blood glucose values.

According to one aspect of the invention, when estimating blood glucose values based on finger blood glucose values, the second period of time can be extended.

According to one aspect of the invention, the closed-loop artificial pancreas system further comprises a preset blood glucose threshold, and different infusion strategies based on the comparison relationship between finger blood glucose values and the blood glucose threshold.

According to one aspect of the invention, the infusion strategy includes administering a high dose, reducing insulin infusion, and pausing insulin infusion.

According to one aspect of the invention, if meals are taken during the second period, at least one finger blood glucose value before the meal and at least one finger blood glucose value after the meal are provided, and the dietary high-dose before the meal is calculated based on the finger blood glucose value before the meal, and the dietary high-dose after the meal is calculated based on the finger blood glucose value after the meal, respectively.

According to one aspect of the invention, the finger blood glucose value before the meal is provided 0-20 minutes before the meal, and the finger blood glucose value after the meal is provided 0-150 minutes after the meal.

According to one aspect of the invention, the calculation of estimated blood glucose value and estimated insulin infusion volume value can be repeated during the second period of time.

According to one aspect of the invention, if the program mechanism still does not receive the real-time blood glucose value after the second period of time, the closed-loop artificial pancreas system switches to the third mode for the third period of time.

According to one aspect of the invention, in the third mode, the insulin infusion amount is 0~30% of the estimated insulin infusion volume value.

According to one aspect of the invention, in the third mode, the insulin infusion amount is 0~10% of the estimated insulin infusion volume value.

According to one aspect of the invention, the third period of time is from 0 to 48 hours.

According to one aspect of the invention, the first period of time is 0-24 hours.

According to one aspect of the invention, the second period of time is from 0 to 48 hours.

According to one aspect of the invention, when the closed-loop artificial pancreas system is in the second mode or the third mode, if the program mechanism recovers to receive the real-time blood glucose value, the closed-loop artificial pancreas system switches to the first mode.

Compared with the prior art, the technical solution of the invention has the following advantages:
The closed-loop artificial pancreas system for safe infusion disclosed in the invention can continue to calculate the estimated insulin infusion volume value required by the patient based on historical blood glucose values and finger blood glucose values during a long period of missing real-time blood glucose value, and inject an appropriate amount of insulin to the patient. It will not inject an inappropriate amount of insulin or even interrupt insulin infusion due to the lack of real-time blood glucose value, avoiding life-threatening situations for the patient and benefiting the patient's treatment.

Further, after inputting finger blood glucose values, the duration of the learning mode can be extended, providing patients with more time to recover and receive real-time blood glucose values without affecting their blood glucose control, which is beneficial for their treatment.

Further, in the closed-loop artificial pancreas system, a preset blood glucose threshold is set, and the input finger blood glucose value is compared with the blood glucose threshold. Based on the comparison results, different insulin infusion strategies are implemented, including high-dose calibration, reduced insulin infusion, and suspension of insulin infusion, which can optimize blood glucose control and benefit patient treatment.

Further, an insulin infusion scheme is provided for meals during the learning mode, and meals are taken during periods of real-time blood glucose deficiency. The closed-loop artificial pancreas system can also administer an appropriate amount of insulin to patients to avoid high blood glucose values that may affect their health, which is beneficial for their treatment.

Further, when the learning mode lasts for a long time, the closed-loop artificial pancreas system will enter a safe mode. After the real-time blood glucose value is missing for a long time, the estimated blood glucose value may deviate uncontrollably from the actual blood glucose value. When the deviation is too large, the estimated insulin amount calculated based on the estimated blood glucose value will not be applicable to the patient. Excessive or insufficient estimated insulin amount may have adverse effects on the patient. After entering a safe mode, the closed-loop artificial pancreas system will administer insulin to the patient according to a predetermined ratio to ensure basic blood glucose control, which is beneficial for the patient's treatment.

Further, regardless of whether the closed-loop artificial pancreas system is currently in learning mode or safe mode, once it resumes receiving real-time blood glucose values, it will enter normal closed-loop mode. In closed-loop mode, the closed-loop artificial pancreas system can provide more accurate blood glucose monitoring and insulin infusion.

## BRIEF DESCRIPTION OF THE DRAWINGS

FIG.1 is a schematic diagram of the module relationship of the general closed-loop artificial pancreas insulin infusion control system according to the embodiment of the invention.

FIG.2 is a schematic diagram of the integrated continuous glucose monitoring device according to an embodiment of the invention.

FIG.3 is a schematic diagram of the split continuous glucose monitoring device according to an embodiment of the invention.

FIG.4a is a schematic diagram of the integrated insulin pump according to an embodiment of the invention.

FIG.4b is a schematic diagram of the split insulin pump according to an embodiment of the invention.

FIG. 5a - 5d show schematic diagrams of the operating environment of the existing management systems for blood glucose management and insulin infusion according to an embodiment of the invention.

FIG. 6 is a schematic diagram of the operating environment of the management systems for blood glucose management and insulin infusion according to an embodiment of the invention.

FIG. 7 is a schematic diagram of the operating environment of the management systems for blood glucose management and insulin infusion according to another embodiment of the invention.

FIG. 8 is a flowchart of a software or firmware update on a medical device according to an embodiment of the invention.

FIG. 9a and FIG. 9b show schematic diagrams of the interface of an APP in use according to an embodiment of the invention.

FIG. 9c and FIG. 9d show schematic diagrams of the interface of an APP in idle according to an embodiment of the invention.

FIG. 10 is a schematic diagram of the safe infusion process of a closed-loop artificial pancreas system according to an embodiment of the invention.

## DETAILED DESCRIPTION

As mentioned above, when the existing closed-loop artificial pancreas system loses the real-time blood glucose value for a long time, it often uses the method of reducing the fixed proportion of insulin infusion and suspending insulin infusion to spend this time. These methods cannot meet the patients' diabetes treatment, and may even put the patients in danger of life.

In order to solve this problem, the invention discloses a closed-loop artificial pancreas system for safe infusion, during a long period of real-time blood glucose value loss, the closed-loop artificial pancreas system can enter a learning mode or a safe mode. Based on historical blood glucose values and finger blood glucose values, it continues to calculate the required insulin infusion amount for the patient and infuses an appropriate amount of insulin to the patient. It will not infuse an inappropriate amount of insulin or even interrupt insulin infusion due to the lack of real-time blood glucose value, avoiding life-threatening situations for the patient and benefiting the patient's treatment.

Various exemplary embodiments of the invention will now be described in detail with reference to the drawings. The relative arrangement of the components and the steps, numerical expressions and numerical values set forth in the embodiments are not to be construed as limiting the scope of the invention.

In addition, it should be understood that, for ease of description, the dimensions of the various components shown in the figures are not necessarily drawn in the actual scale relationship, for example, the thickness, width, length or distance of certain units may be exaggerated relative to other parts.

The following description of the exemplary embodiments is merely illustrative, and is not intended to be in any way limiting the invention and its application or use. The techniques, methods, and devices that are known to those of ordinary skill in the art may not be discussed in detail, but such techniques, methods, and devices should be considered as part of the specification.

It should be noted that similar reference numerals and letters indicate similar items in the following figures. Therefore, once an item is defined or illustrated in a drawing, it will not be discussed further in the following description of the drawings.

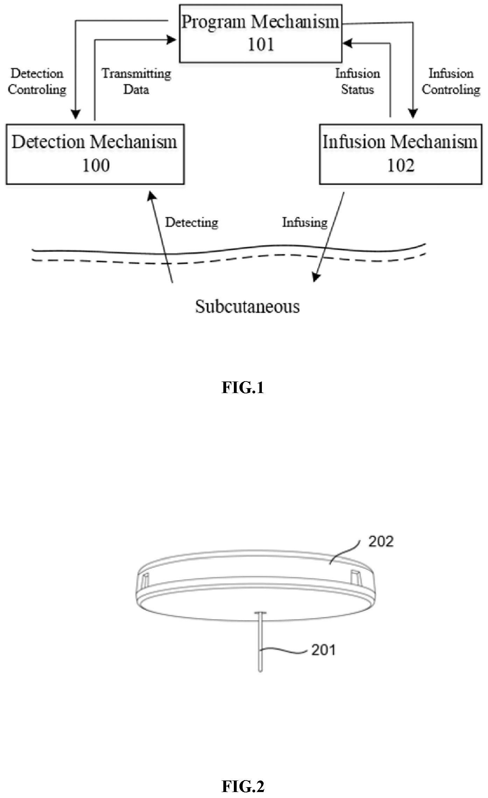

FIG.1 is a schematic diagram of the module relationship of the closed-loop artificial pancreas insulin infusion control system according to the embodiment of the invention.

The closed-loop artificial pancreas insulin infusion control system disclosed in the embodiment of the invention mainly includes a detection mechanism 100, a program mechanism 101, and an infusion mechanism 102.

The detection mechanism 100 is used to continuously detect the user's real-time blood glucose (BG) level. Generally, detection mechanism 100 is a Continuous Glucose Monitoring (CGM) for detecting real-time BG, monitoring BG changes, and sending them to the program mechanism 101. The CGM includes an implantable sensor, the sensor is connected to a transmitter, the transmitter further includes a memory, a processor, a communication interface, and the like, and the transmitter is used for transmitting at least information about the blood glucose value monitored by the CGM, as well as information about an identifier of the CGM, and the like.

The infusion mechanism 102 includes the essential mechanical assemblies and electronic control unit used to infuse insulin, such as reservoir, drive structures, fluid path and infusion needles, power supplies, circuit boards, and so on, and is controlled by program mechanism 101. Generally, the infusion mechanism 102 is an insulin pump, and the electronic control unit includes a memory, a processor, a communication interface, etc. According to the current insulin infusion dose sent by program mechanism 101, infusion mechanism 102 injects the current insulin dose required into the user's body. At the same time, the real-time infusion status of infusion mechanism 102 can also be fed back to program mechanism 101.

Program mechanism 101 is used to control the detection mechanism 100 and the infusion mechanism 102. Therefore, program mechanism 101 is connected to detection mechanism 100 and infusion mechanism 102, respectively. Here, the connection refers to a conventional electrical connection or a wireless connection.

The embodiment of the invention does not limit the specific positions and connection relationships of the detection mechanism 100, the program mechanism 101 and the infusion mechanism 102, as long as the aforementioned functional conditions can be satisfied.

As in an embodiment of the invention, the three are electrically connected to form a single part. Therefore, the three mechanisms can be attached on only one position of the user's skin. If the three mechanisms are connected as a whole and attached in only one position, the number of the device on the user skin will be reduced, thereby reducing the interference of more attached devices on user activities. At the same time, it also effectively solves the problem of poor wireless communication between separating devices, further enhancing the user experience.

Another embodiment of the invention is that the program mechanism 101 and the infusion mechanism 102 are electrically connected to form a single part, while the detection mechanism 100 is separately provided in another part. At this time, the detection mechanism 100 and the program mechanism 101 transmit wireless signals to realize the mutual connection. Therefore, program mechanism 101 and infusion mechanism 102 can be attached to the user's skin position while the detection mechanism 100 is attached to the other position.

Another embodiment of the invention is that the program mechanism 101 and the detection mechanism 100 are electrically connected, forming a single part, while the infusion mechanism 102 is separately provided in another part. The infusion mechanism 102 and the program mechanism 101 transmit wireless signals to realize the mutual connection. Therefore, program mechanism 101 and the detection mechanism 100 can be attached to the same position of the user's skin while the infusion mechanism 102 is attached to the other position.

Another embodiment of the invention is that the three are provided in different parts, thus being attached to different positions. Simultaneously, program mechanism 101, detection mechanism 100, and infusion mechanism 102 transmit wireless signals to realize the mutual connection.

Another embodiment of the invention is that the three are provided in different parts, the detection mechanism 100 and the infusion mechanism 102 are attached to different locations of the user's skin, respectively, while the program mechanism 101 does not have to be attached to the skin, and control the detection mechanism 100 and the infusion mechanism 102 by means of a handheld or portable device. At this time, the program mechanism 101 transmits wireless signals to the detection mechanism 100 and the infusion mechanism 102, respectively, to achieve connection with each other.

The wireless connections described in the foregoing embodiments include, but are not limited to, radio frequency (RF) communications (e.g., radio frequency identification (RFID), Zigbee communication protocol, WiFi, infrared, wireless Universal Serial Bus (USB), Ultra-Wide Band (UWB), Bluetooth® communication protocol, and cellular communications, such as Code Division Multiple Access (CDMA) or the Global System for Mobile Communications (GSM).

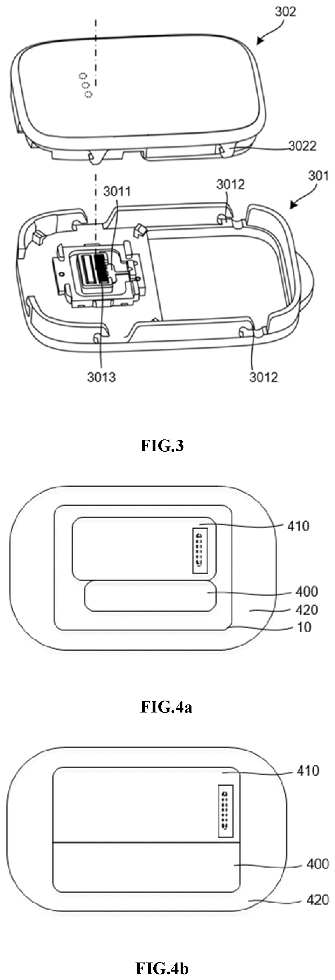

FIG.2 is a schematic diagram of the integrated continuous glucose monitoring device according to an embodiment of the invention. FIG.3 is a schematic diagram of the split continuous glucose monitoring device according to an embodiment of the invention.

The CGM comprising a sensor and a transmitter, mounted on the patient by means of an auxiliary mount, pierced subcutaneously. The sensor is used to collect analyte data in the human body and transmit the collected analyte content information. The transmitter is connected to the sensor, and it is used to receive the information on the blood glucose value, which is transmitted by the sensor that has been implanted under the skin, and convert the information into a wireless signal for outputting. Each CGM has a unique identifier including, but not limited to a device identifier, a hardware identifier, a generic unique identifier, a serial number, an identifier based on communication protocol (e.g., a BLE ID), a manufacturer's identifier, and the like, preferably, the identifier is formed by a plurality of randomly-combined numerical and alphabetic, and can be set on a housing or package of the CGM, or may also be set differently depending on the type of the CGM.

FIG.2 is a schematic diagram of the integrated continuous glucose monitoring device, i.e., the sensor and the transmitter have been integrated together prior to use, and is a single-use product, which is discarded after use, as shown in FIG. 2. The integrated continuous glucose monitoring device includes a sensor 201, a housing 202, and a transmitter (not shown in the figure) set inside the housing 202, the sensor 301 is used to monitor the patient's body fluid blood glucose value information, and transmit the said blood glucose value information to the transmitter through an internal circuit, which in turn sends it to a receiver. The identifier may be provided on the outer housing of the CGM or on the outer packaging or within the CGM.

FIG.3 is a schematic diagram of the split continuous glucose monitoring device, i.e., the sensor and the transmitter are two different parts prior to use, packaged separately, and only integrated together when in use, as shown in FIG. 3. The split continuous glucose monitoring device includes a base 301 and a transmitter 302, the base is provided with a sensor 3011, the transmitter 302 has a separate housing, the housings of the base 301 and the transmitter 302 are respectively provided with snap-in structures 3012 and 3022, the base 301 and the transmitter 302 are snapped into a whole through the snap-in structures when in use, the sensor 3011 is electrically connected to the transmitter 302 through the electrical connection 3013, the sensor 301 is used to monitor the patient's blood glucose value information, the blood glucose value information is transmitted to the transmitter 302 through the electrical connection 3013, and then sent to the receiver by the transmitter 302.

In one embodiment of the invention, both the sensor and the transmitter of the split continuous glucose monitoring device are single-use products, which are discarded after use, and therefore the identifier may be provided on the housing or the outer packaging of the sensor or the transmitter. In yet another embodiment of the invention, only the sensor of the split continuous glucose monitoring device is a single-use product and the transmitter is a reusable product, therefore, preferably, in this embodiment, the identifier is provided on the housing or the outer packaging of the transmitter, which reduces the frequency of tying the patient's information to the identifier and improves the patient experience. This will be described in more detail below.

When the identifier is set on the housing or outer packaging of the CGM or transmitter, it is set in a form that includes, but is not limited to, a QR code, a bar code, or an NFC tag.

FIG.4a is a schematic diagram of the integrated insulin pump according to an embodiment of the invention. FIG.4b is a schematic diagram of the split insulin pump according to an embodiment of the invention.

In this embodiment of the invention, the insulin pump is a patch-type insulin pump, i.e., an insulin pump that does not comprise a long catheter, includes an infusion mechanism and a control mechanism, and being integrally affixed to the surface of the user's skin by an adhesive patch, and the medication is infused directly from the reservoir to the subcutaneous area along the infusion needle.

Each insulin pump has a unique identifier including, but not limited to a device identifier, a hardware identifier, a generic unique identifier, a serial number, an identifier based on communication protocol (e.g., a BLE ID), a manufacturer's identifier, and the like, preferably, the identifier is formed by a plurality of randomly-combined numerical and alphabetic, and can be set on a housing or package of the insulin pump, or may also be set differently depending on the type of the insulin pump.

FIG.4a is a schematic diagram of the integrated insulin pump, i.e., the infusion mechanism 410 and the control mechanism 400 of the insulin pump are provided inside the same housing 10, and both are connected by wires and are affixed to a location on the user's skin by means of an adhesive patch 420, which is disposed of in its entirety after a single use. an identifier may be provided on the outer housing of the insulin pump or on the outer packaging or inside the insulin pump.

FIG.4b is a schematic diagram of the split insulin pump, i.e., the infusion mechanism 410 and the control mechanism 400 of the insulin pump are provided in two different housings, both of which are connected by waterproof plugs or directly snap together and electrically connected as a whole, an identifier may be provided on the outer housing of the infusion mechanism and/or the control mechanism or on the outer packaging or inside the insulin pump.

In one embodiment of the invention, both the infusion mechanism and the control mechanism of the split insulin pump are single-use products, which are discarded after use, and therefore the identifiers may be provided on the housing or on the outer packaging of the infusion mechanism and/or the control mechanism. In yet another embodiment of the invention, only the infusion mechanism of the split insulin pump is a single-use product, while the control mechanism is a reusable product, and therefore, preferably, in this embodiment, the identifier is set on the housing of the control mechanism or on the outer packaging, which can reduce the frequency of binding of patient information and identifiers, and improve the patient experience. This is described in more detail below.

When the identifier is set on the housing of the control mechanism or on the outer packaging, it is set in a form that includes, but is not limited to, a QR code, a bar code, or an NFC tag.

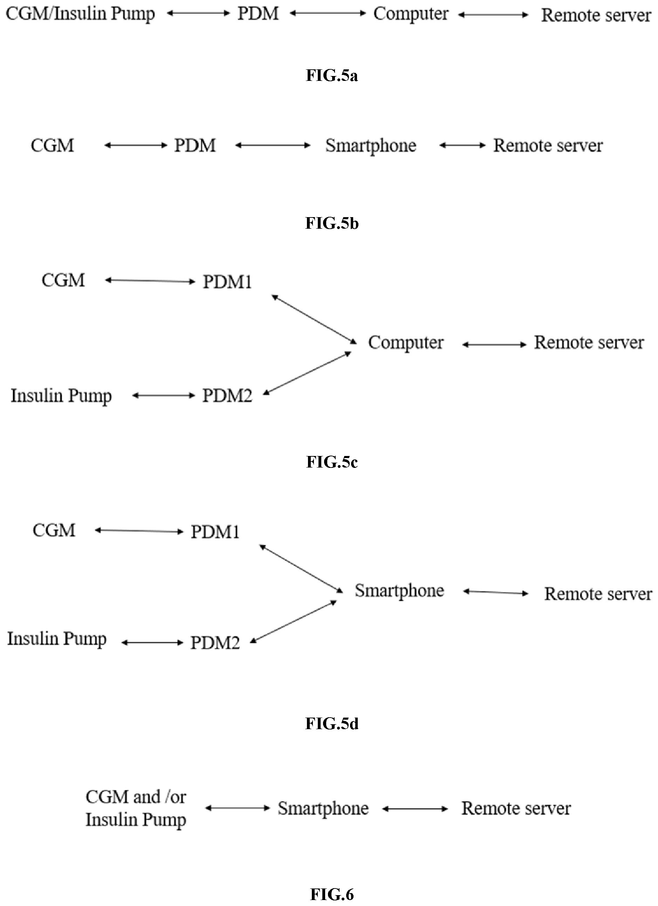

FIG. 5a - 5d show schematic diagrams of the operating environment of the existing management systems for blood glucose management and insulin infusion according to an embodiment of the invention.

The program mechanism used to control the CGM or insulin pump is typically a personal dedicated handheld device (PDM), as shown in Fig. 5a. Because PDM is typically designed as low-power devices that can only communicate with other devices via communication methods such as Bluetooth or NFC, when a CGM or insulin pump needs to be updated, the manufacturer typically sends the latest installation package to the computer via a remote server and authorizes the user to manually download the update installation package, and connect the CGM and/or insulin pump to the computer to start and complete the update. This process may be particularly cumbersome for users who are unfamiliar or uncomfortable with modifying rules of their CGM and/or insulin pump. As a result, users may avoid updating and miss important bug fixes, security updates, and added features. On this basis, Abbott has upgraded the update method of CGM, allowing users to update CGM through smartphones. Specifically, the manufacturer will send the latest installation package to the individual's smartphone through a remote server, the smartphone is connected to the PDM, and the smartphone will send the update information to the PDM, which will then control the CGM to complete the update, although this method can simplify the update process to some extent by eliminating the step to complete the update through the computer, it still requires an intermediary medium, the smartphone, to connect to the remote server and the PDM, and ultimately the PDM controls the CGM to complete the update, which is still a relatively cumbersome process, and the user may still avoid the update, thus missing important bug fixes, security updates, and feature additions.

At present, Abbott has upgraded the update method of CGM, as shown in FIG. 5b, allowing users to update CGM through smartphones. Specifically, the manufacturer will send the latest installation package to the individual's smartphone through a remote server, the smartphone is connected to the PDM, and the smartphone will send the update information to the PDM, which will then control the CGM to complete the update, although this method can simplify the update process to some extent by eliminating the step to complete the update through the computer, it still requires an intermediary medium, the smartphone, to connect to the remote server and the PDM, and ultimately the PDM controls the CGM to complete the update, which is still a relatively cumbersome process, and the user may still avoid the update, thus missing important bug fixes, security updates, and feature additions.

At the same time, due to the inconsistency between the manufacturers of CGM and insulin pumps, the dedicated PDMs for each may not compatible with the other, so different PDM1 and PDM2 are usually required to control CGM and insulin pumps respectively, as shown in Fig. 5c, which further causes inconvenience to the users. When a software or firmware in a CGM or insulin pump needs to be updated, users are more likely to avoid the update. If the update is done via the Abbott method, as shown in FIG. 5d, not only is there the issue that one PDM can't able to control both the CGM and insulin pump, but also there is another issue that more intermediary medias to complete the update are required, and the user is still likely to avoid the update, thus missing out on important bug fixes, security updates, and feature additions.

FIG. 6 is a schematic diagram of the operating environment of the management systems for blood glucose management and insulin infusion according to an embodiment of the invention.

As shown in FIG. 6, the management system for blood glucose monitoring and insulin infusion includes at least one wearable medical device, such as a CGM and/or an insulin pump, and a mobile device, such as a smartphone, before the CGM and/or the insulin pump are mounted on the surface of the skin of the user, respectively, the user can search for and download a dedicated APP for controlling the CGM and/or the insulin pump in an app store of the smartphone, and the user can create a new account on the dedicated APP to pair the patient's personal information with the information of the CGM to be worn and/or the information of the insulin pump to be worn, so as to pair the smartphone with the medical devices. The user may be the patient himself, a healthcare provider or a guardian, and the personal information of the patient includes name, age, gender, cell phone number, etc., and the information of the CGM and/or insulin pump worn includes the identifier information of the CGM and/or insulin pump. At the same time, the smartphone uploads the personal information of the patient and the identifier information of the CGM and/or the insulin pump to a remote server, which can store the information uploaded by the smartphone and verify whether the identifier information of the CGM and/or the insulin pump is valid, and if a certain identifier information already exists in the remote server, the remote server sends an alert to the smartphone to remind the user that the CGM or insulin pump has been used and needs to be replaced. When the CGM and/or the insulin pump is mounted on the skin of the patient and successfully activated, the CGM and/or the insulin pump starts to work, the transmitter of the CGM sends the monitored blood glucose information to the smartphone and further uploads it to the remote server, and the control mechanism of the insulin pump receives the insulin infusion information and controls the infusion mechanism to infuse the insulin, and at the same time sends the infusion status to the smartphone and further uploads it to the remote server.

It should be noted that the CGM and insulin pump in the embodiment of the invention are developed and produced by the same manufacturer, and thus can be controlled by the same dedicated APP in the smartphone. Assuming that CGM or insulin pumps produced by other manufacturers also can be controlled by a dedicated APP in the smartphone, and the inconvenience to the user due to the use of different APPs for controlling the CGM and the insulin pump respectively can be avoided, and the user experience can be improved.

When the CGM and/or insulin pump worn by the patient needs to be replaced for reasons such as reaching the end of its life cycle or failing, the unique identifier information of the new CGM and/or insulin pump also needs to be updated by pairing with the patient's personal information via the smartphone and further uploaded to a remote server. The patient's personal information is entered manually, and the identifier information of the CGM and/or insulin pump may also be entered manually or by scanning a QR code, a barcode, or NFC tags on the housing or on the outer packaging of the CGM and/or the insulin pump.

When the CGM is split and the transmitter is reusable, the identifier of the CGM is set on the housing or packaging of the transmitter, so that the patient only needs to replace the sensor when replacing the CGM without replacing the transmitter, and the identifier of the CGM remains unchanged, and thus there is no need for updating the pairing of the identifier of the CGM with the personal information of the patient via a smartphone, and no need for uploading it to a remote server, and thus the number of operational steps can be reduced to improve the patient experience.

When the insulin pump is split and the control mechanism is reusable, the identifier of the insulin pump is set on the housing or packaging of the control mechanism, and the patient only needs to change the infusion mechanism when replacing the insulin pump without changing the control mechanism, and the identifier of the insulin pump remains unchanged, and thus there is no need for updating the pairing update of the identifier of the insulin pump with the personal information of the patient via the smartphone or in uploading it to a remote server, and thus the operational steps can be reduced and the patient experience can be improved.

The smartphone and the CGM and/or insulin pump, and the remote server communicate with each other via wireless communication, which may be via, for example, but not limited to, radio frequency (RF) communication (e.g., radio frequency identification (RFID), Zigbee communication protocols, WiFi, infrared, wireless Universal Serial Bus (USB), Ultra-Wide Band (UWB), Bluetooth® communication protocols, and cellular communication, such as Code Division Multiple Access (CDMA) or Global System for Mobile Communications (GSM). communications, such as Code Division Multiple Access (CDMA) or Global System for Mobile Communications (GSM). Preferably, the smartphone and the remote server communicate with each other via WiFi and/or cellular communication, and the smartphone and the CGM and/or insulin pump communicate with each other via Bluetooth® communication protocol.

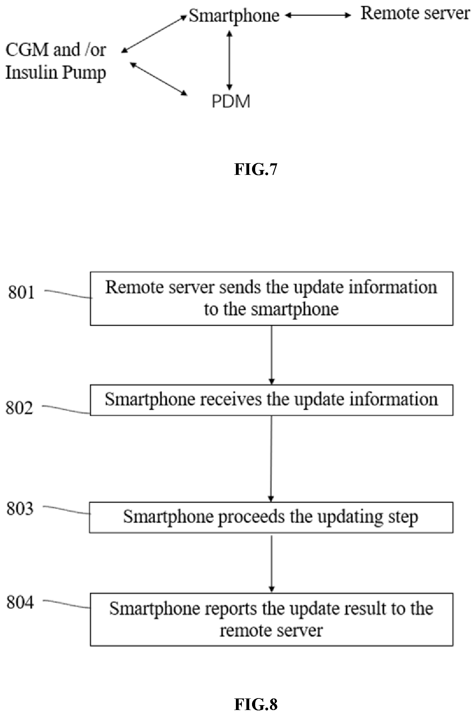

FIG. 7 is a schematic diagram of the operating environment of the management systems for blood glucose management and insulin infusion according to another embodiment of the invention.

As shown in FIG. 7, the management system for blood glucose monitoring and insulin infusion includes a dedicated handheld device, PDM, which communicates with a CGM and/or insulin pump, a smartphone, via short-range communication such as Bluetooth, and the PDM pairs with the CGM and/or insulin pump in a manner similar to that of the aforementioned pairing of the smartphone with CGM and/or insulin pump, whereby the user creates a new account on the PDM and pairs the patient's personal information with the identifier information of the CGM and/or insulin pump to be worn. The user may also be the patient himself, a healthcare provider or a guardian, preferably, in one embodiment of the invention, the PDM and the smartphone are used by different persons e.g., the PDM is used by the patient himself, and the smartphone is used by a guardian. When the patient is an elderly person or a young child, or a diabetic patient with a special disease who is unable to perform self-monitoring of blood glucose and/or insulin infusion, the guardian can limit the patient's operation on the PDM through the lock mode on the smartphone's dedicated APP, such as making it impossible for the patient to view real-time blood glucose value information on the PDM, receive blood glucose alarms, change the alarm settings, modify the insulin pump's insulin infusion settings, unable to pause or stop insulin infusion, etc. It can prevent patients from mis-operation of PDM, such as deleting the device, disengaging the connection between PDM and CGM, insulin pump, which can affect the normal glucose monitoring of CGM and insulin infusion of insulin pump. not receiving the blood glucose alarms, it can also prevent the blood glucose alarms from interfering with or affecting the patients.

It should be noted that the CGM and the insulin pump in the embodiment of the invention are developed and produced by the same manufacturer, and thus can be controlled by the same PDM, which can avoid the inconvenience caused to the user due to the use of different PDMs for controlling the CGM and the insulin pump respectively, and improve the user experience.

FIG. 8 is a flowchart of a software or firmware update on a medical device according to an embodiment of the invention.

After the manufacturer develops a new software or firmware for CGM and/or insulin pump, it creates an update project and uploads it to the remote server. The update project includes, at least, the uploading of the new software or firmware installation package, information about the identifiers of the CGMs and/or insulin pumps that the software or firmware need to be updated, the version of the updated software or firmware, and information about whether the update is mandatory or not.

Step 801, the remote server sends the update information of the CGM and/or insulin pump to the smartphone.

Specifically, the remote server sends the software or firmware update information to the smartphones which is matched with the CGM and/or insulin pump that need to update the software or firmware according to the update item uploaded by the manufacturer. In one embodiment of the invention, the update information is sent directly to a dedicated APP for controlling the CGM and/or insulin pump. in another embodiment of the invention, the update information is sent to an application store of the smartphone.

Step 802: The smartphone receives the update information of the CGM and/or insulin pump.

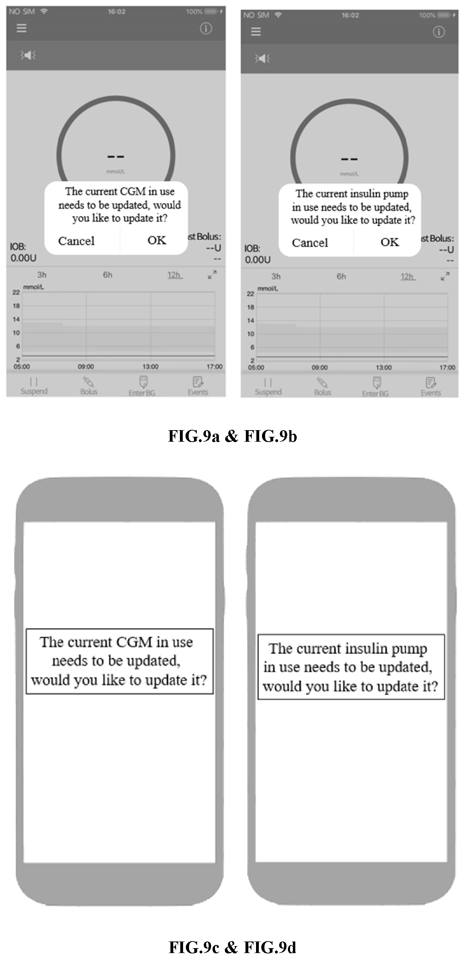

When the remote server sends the update information to the dedicated APP, if the dedicated APP is in use, the update information will be displayed in the form of text or other forms showing "the currently CGM in use needs to be updated, would you like to update" or "the currently insulin pump in use needs to be updated, would you like to update", as shown in FIG. 9a or FIG. 9b ", and the user receives the update message by clicking "OK". If the dedicated APP is in idle, a notification in the background will show "the currently CGM in use needs to be updated, would you like to update" or "the currently insulin pump in use needs to be updated, would you like to update", as shown in FIG. 9c or FIG. 9d, and then click the notification to enter the dedicated APP, and then the screen shown in FIG. 9a or FIG. 9b is displayed, and the user receives the update information by clicking "OK". If the user does not respond within a certain period of time, such as one minute, or 30 seconds, the notification will be sent at a predetermined frequency, such as every five minutes, or with the extension of time, the interval between the notification is gradually longer until the user responds.

When the remote server sends the update information to the app store, when the app store is in use or in idle, the update information is displayed in a form similar to that displayed by the dedicated app, which will not be repeated here.

At step 803, the smartphone proceeds the updating step.

The updating step includes, but is not limited to, deleting the original application, downloading and installing a new installation package, program upgrading or downgrading, such as upgrading or downgrading to a version of the software or firmware of the CGM and/or insulin pump that is set by the manufacturer in the updating program.

In one embodiment of the invention, the smartphone automatically starts the updating step when the user receives and confirms the updating information via a dedicated APP or an application store. If the updating step is interrupted due to signal interruption or other reasons during the updating process, the smartphone can remind the user by SMS, vibration, ringing, light flashing, and other visual, auditory, sensory and other means, and remind the user whether to enter the updating step again after the signal is restored or the other malfunction is solved, and the user clicks on the confirmation to enter the updating step once again until the updating step is completed.

In another embodiment of the invention, after the signal is restored or the other malfunction is solved, the smartphone directly enters the updating step again, no user confirmation is required first.

Step 704, the smartphone reports the update result to the remote server.

After the update is completed, the smartphone connects to the CGM and/or the insulin pump via wireless communication, and if the connection is successful within a certain period of time, e.g., 2 minutes, the smartphone uploads the result of the successful update to the remote server, and the contents of the upload include the identifier information of the CGM and/or the insulin pump, the version of the software or firmware for which the update has been completed, the time at which the update has been completed, and the like. If the uploading of the update record to the remote server is temporarily unsuccessful due to remote communication, etc., the record is saved locally and uploaded to the remote server again when remote communication is resumed until the upload is successful. If the connection is not successful within a certain period of time, such as 2 minutes, which indicated the update is failed, and the smartphone uploads the result of the failed update to the remote server, and the uploaded result includes at least the identifier information of the CGM and/or the insulin pump, the version of the software or firmware that is ready to be updated to, the time of the failed update, and the like.

Therefore, the total number of CGMs and/or insulin pumps that need to be updated, the identifier information and the number of CGMs and/or insulin pumps for which the update was successful and for which the update failed can be viewed in the update project of the remote server. The remote server can pause the updating for projects that have already started updating, and the remote server sends update notifications to the smartphone again at a certain frequency for projects with failed updates.

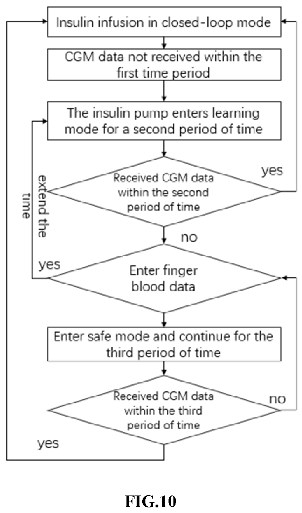

Referring to Fig. 10, Fig. 10 is a schematic diagram of the safe infusion process of the closed-loop artificial pancreas system according to an embodiment of the invention.

In the embodiment of the invention, the program mechanism 101 can calculate the insulin infusion volume on the premise of continuously obtaining the real-time blood glucose value of the detection mechanism 100, and the normal insulin infusion is carried out by the infusion mechanism 102, which is the first mode of the closed-loop artificial pancreas system, that is, the closed-loop mode. Once the communication between the detection mechanism 100, the program mechanism 101 and the infusion mechanism 102 is abnormal, or the sensor of the detection mechanism 100 is damaged, and the program mechanism 101 cannot receive the real-time blood glucose value of the detection mechanism 100 for a long time, the closed-loop artificial pancreas system will exit the closed-loop mode. If the patient does not set the insulin infusion rate, or the sensor is damaged and cannot be replaced with a new sensor, the infusion mechanism 102 will stop insulin infusion, which will have a serious adverse impact on the health of diabetes patients, and is not conducive to the treatment of diabetes patients.

Based on the above usage scenarios, the closed-loop artificial pancreas system also requires a safety guarantee scheme where the program mechanism 101 can accurately calculate the insulin infusion amount and the infusion mechanism 102 can complete the insulin infusion when real-time blood glucose value from the detection mechanism 100 cannot be obtained for a long time, in order to avoid adverse effects on the patient's health.

In the embodiment of the invention, under normal circumstances, the program mechanism 101 continuously receives the patient's blood glucose value from the detection mechanism 100 at fixed time intervals, such as 2 minutes. The program mechanism 101 calculates the insulin infusion amount and the infusion mechanism 102 completes the infusion. If the program mechanism 101 accidentally does not receive real-time blood glucose value from the detection mechanism 100 at one or several times intervals, the program mechanism 101 can continue to calculate the insulin infusion amount based on the last blood glucose value from the detection mechanism 100. However, such calculation cannot continue for a long time because the patient's blood glucose may undergo significant changes after a long time.

In the embodiment of the invention, when the closed-loop artificial pancreas system does not acquire the real-time blood glucose value of the detection mechanism 100 during the continuous t1 time period, the closed-loop artificial pancreas system will enter the second mode, that is, the learning mode. In learning mode, program mechanism 101 predicts the estimated blood glucose value for the next t2 time period based on recorded historical blood glucose values, and calculates the estimated insulin level for the t2 time period based on the estimated blood glucose value. Insulin infusion during the t2 time period is completed by infusion mechanism 102 to ensure the patient's blood glucose safety during the t2 time period.

In the embodiments of the invention, the average value of real-time blood glucose value from a period before it is missing, such as the average value of 50 data points (corresponding to 100 minutes), or based on the speed and acceleration of real-time blood glucose changes, or combined with the patient's historical data, or using more complex prediction models, can be used to calculate the estimated blood glucose value.

In the embodiment of the invention, the duration t1 during which the closed-loop artificial pancreas system cannot receive real-time blood glucose value and enters the learning mode can be set to 0-24 hours. After 24 hours, if the closed-loop artificial pancreas system still does not receive real-time blood glucose value from the detection mechanism 100 or estimated blood glucose value calculated by the program mechanism 101, the closed-loop artificial pancreas system will stop insulin infusion, affecting the patient's treatment.

In the embodiment of the invention, the learning mode, that is, the t2 time period for calculating the estimated blood glucose value, can be set from 0 to 48 hours. Since the start time of t2 time period is at the end of t1 time period, setting t2 t time period too long may cause a significant deviation between the estimated blood glucose value and the real-time blood glucose value. Therefore, the estimated insulin level calculated based on the estimated blood glucose value may be dangerous for patients.

In the embodiment of the invention, the estimated infusion rate $V_t$ of the infusion mechanism 102 during the t2 time period can be obtained by the following equation:

$$V_{t2} = \frac{G_{t2} - G_{tar}}{ISF * t} \qquad (1)$$

wherein

t is the time period for each calculation cycle.

$V_t$ is the estimated infusion rate during t time period.

$G_t$ is the estimated blood glucose value during t time period.

$G_{tar}$ is the preset target blood glucose value.

ISF is the insulin sensitivity of patients.

During the t2 time period of the learning mode, the above calculation is completed in program mechanism 101, and insulin infusion mechanism 102 performs insulin infusion according to the estimated infusion rate.

In the embodiment of the invention, if the t time period is set to be short, the calculation of insulin infusion rate based on equation (1) can be repeated during the time period when real-time blood glucose value is missing. For example, if the t time period is set to 10 minutes, after insulin infusion at the estimated infusion rate for 10 minutes, the estimated blood glucose value Gt can be re determined, and the estimated infusion rate for the next 10 minutes can be calculated again based on equation (1) to complete insulin infusion until the closed-loop artificial pancreas system resumes receiving real-time blood glucose value. When estimating the infusion rate through repeated calculations, the estimated blood glucose value used comes from a secondary calculation of the estimated blood glucose value in the previous round of calculations, and the calculation method is consistent with the calculation method of the estimated blood glucose value in the previous round.

Obviously, such repeated calculations have limitations. As time increases, the deviation between the estimated infusion rate and the actual infusion rate required by the patient gradually becomes uncontrollable. When the deviation is too large, excessive or insufficient insulin infusion is dangerous for the patient.

In addition, if only estimated blood glucose values are used to calculate the estimated infusion rate, during periods of missing real-time blood glucose value, it may not be sufficient to meet the precise insulin infusion requirements, which can still affect the patient's treatment outcome.

In the embodiments of the invention, in order to improve the insulin infusion accuracy of the closed-loop artificial pancreas system during periods of missing real-time blood glucose value, the estimated infusion rate or estimated blood glucose value can also be calculated by combining the patient's finger blood glucose (BG) level. When calculating the estimated blood glucose value Gt, taking into account both the finger blood glucose value BG and the real-time blood glucose value lost in the previous period, the weighted average method is used to calculate the estimated blood glucose value $G_t$. As the calculation time increases, the weight of the finger blood glucose value BG can be dynamically increased, making it more reliable to trust the finger blood glucose value BG.

In the embodiment of the invention, when the patient measures the finger blood glucose value BG and inputs it into the closed-loop artificial pancreas system, the duration of the learning mode can be extended because as the finger blood glucose value BG is added to the calculation of the estimated blood glucose value Gt, the estimated blood glucose value Gt will become more reliable, and the calculated estimated insulin infusion rate $V_t$ will also be safer. Each time the patient inputs the finger blood glucose value BG, the learning mode can be extended for 0-48 hours, providing more available time for the patient to take measures to recover and receive real-time blood glucose value without affecting the patient's treatment.

In the embodiments of the invention, when the finger blood glucose value BG of the patient is at different levels, the closed-loop artificial pancreas system can adopt different infusion strategies to achieve higher accuracy and better adapt to the patient's infusion mode. In equation (1), during the learning mode, it is necessary to calculate the estimated infusion rate by combining the patient's preset blood glucose threshold, which includes the target blood glucose value $G_{tar}$ and the high and low blood glucose thresholds. The target blood glucose value $G_{tar}$ is used to distinguish the level of finger blood glucose value BG, and the infusion strategy of the closed-loop artificial pancreas system is determined by comparing the finger blood glucose value BG and the target blood glucose value $G_{tar}$.

In the embodiments of the invention, the target blood glucose value $G_{tar}$ can be determined by the patient or their medical staff, and input and stored in a closed-loop artificial pancreas system. The target blood glucose value $G_{tar}$ can be dynamically adjusted according to the patient's treatment status to achieve the best treatment effect for the patient. In the descriptive implementation of this plan, the target blood glucose value $G_{tar}$ can be set to 5.6mmol/L, 6.1 mmol/L, or 6.7 mmol/L, etc.

In the embodiment of the invention, after the patient inputs the finger blood glucose value BG, the program mechanism 101 automatically compares the finger blood glucose value BG with the target blood glucose value $G_{tar}$. If the finger blood glucose value BG is greater than the target blood glucose value $G_{tar}$, the closed-loop artificial pancreas system needs to inject a high-dose IC for calibration. The calibrated high-dose IC for infusion is determined by the following equation:

$$I_C = \frac{BG - G_{tar}}{ISF} - IOB \qquad (2)$$

wherein
IOB is the amount of ineffective insulin in the patient's body.

In the embodiment of the invention, if the finger blood glucose value BG is less than the target blood glucose value but greater than or equal to 3.9 mmol/L, the closed-loop artificial pancreas system needs to reduce the insulin infusion volume for a period of time. The reduced infusion volume can be achieved by reducing the infusion rate. The insulin infusion rate after reducing the infusion volume is 60% to 95% of the estimated infusion rate. Preferably, insulin infusion is performed at 75% of the estimated infusion rate. During the learning mode period, the duration of reducing insulin infusion rate is 10-120 minutes. Specifically, the time td for reducing infusion rate can be determined by the following equation:

$$t_d = \frac{\Delta I}{\Delta V} \qquad (3)$$

wherein

$\Delta I$ is the reduced insulin infusion volume.
$\Delta V$ is the reduced infusion rate.

In the embodiment of the invention, the reduced insulin infusion amount $\Delta I$ after inputting the finger blood glucose value BG can be determined by the following equation:

$$\Delta I = \frac{G_{tar} - BG}{ISF} \qquad (4)$$

In the embodiment of the invention, if insulin infusion is carried out at 75% of the estimated infusion rate, the reduced infusion rate $\Delta V$ can be determined as 25% of the estimated infusion rate $V_t$. This value is for illustrative purposes only.

In the embodiment of the invention, if the finger blood glucose value BG is less than 3.9mmol/L, the closed-loop artificial pancreas system suspends insulin infusion, and the pause time $t_s$ is determined by the following equation:

$$t_s = \frac{G_{tar} - BG}{ISF * V_t} \qquad (5)$$

After the pause in infusion is completed, the closed-loop artificial pancreas system resumes estimating the infusion rate for infusion, or prompts the patient to remeasure finger blood, and then determines the next infusion strategy based on the remeasured finger blood glucose values.

In some embodiments of the invention, during the duration of the learning mode, if the patient needs to have a meal, this will result in significant fluctuations in blood glucose values for a period of time after the meal. Therefore, before and after the meals, patients need to input their finger blood glucose value BG at least once for the program mechanism 101 to calculate the high-dose $I_F$ of the diet.

Specifically, the patient measures their finger blood glucose value BG once every 0-20 minutes before and 0-150 minutes after the expected meal. During this period, the high-dose $I_F$ of the infused diet can be determined by the following equation:

$$I_F = Carbs * IC + \frac{BG - G_{tar}}{ISF} - IOB \qquad (6)$$

wherein

Carbs is the carbohydrate content of food consumed during meals, g;
IC is the ratio of insulin to carbohydrates.

The high-dose of the patient's diet before and after the meals is determined by the finger blood glucose values BG measured before and after the meals, respectively.

In the embodiment of the invention, after the maximum t2 time period that the learning mode can last, if the closed-loop artificial pancreas system still does not resume receiving real-time blood glucose value, the closed-loop artificial pancreas system will enter the third mode, that is, the safety mode, and last for t3 time period. In safe mode, for the sake of patient safety, the closed-loop artificial pancreas system automatically injects insulin into the patient at a lower rate to maintain the baseline insulin treatment level.

In the embodiment of the invention, in safe mode, the closed-loop artificial pancreas system injects 0-30% of the normal dose of insulin at an estimated infusion rate. The preferred closed-loop artificial pancreas system is used to estimate the infusion rate and administer 0-10% of the normal dose of insulin.

In the embodiments of the invention, the sustainable t3 time period for the safe mode is 0-48 hours. In the safe mode, the amount of insulin infused into the closed-loop artificial pancreas system is relatively small. During this period, the patient may have multiple meals and sleep, and prolonged duration may lead to high blood glucose values, affecting the patient's treatment.

In the embodiment of the invention, if the closed-loop artificial pancreas system has not yet resumed receiving real-time blood glucose values after the sustainable t3 time period of the safe mode ends, it will prompt the patient to input finger blood glucose values BG to extend the duration of the safe mode. Obviously, the duration of the safe mode cannot be repeatedly extended. At this time, the closed-loop artificial pancreas system can also be set to alert patients, urging them to take measures to restore real-time blood glucose values as soon as possible, or to send distress signals to their monitoring personnel, medical staff, sales consultants, manufacturers, etc.

In the embodiment of the invention, if the patient uses the closed-loop artificial pancreas system for less than 7 days, due to the system's inability to record sufficient patient historical data, the maximum t2 time period for the closed-loop artificial pancreas system to enter the learning mode will be shortened after real-time blood glucose value is lost, for example, from 24 hours to 12 hours. In this scenario, the patient is still allowed to input finger blood glucose value and the learning mode duration will be extended, but the extended duration will be shortened to 0-12 hours.

In the embodiments of the invention, regardless of when the closed-loop artificial pancreas system enters the learning mode, if real-time blood glucose value from the detection mechanism 100 is not restored and finger blood glucose value is not input after the learning mode ends, the closed-loop artificial pancreas system will enter the safety mode.

In the embodiment of the invention, in the learning mode, although the patient inputs finger blood glucose value, the closed-loop artificial pancreas system will also enter safe mode after exceeding the allowed delay.

In the embodiments of the invention, regardless of whether the closed-loop artificial pancreas system is currently in learning mode or safe mode, once real-time blood glucose value is restored to normal reception, the closed-loop artificial pancreas system will return to the normal closed-loop mode and calculate and administer insulin based on real-time blood glucose value. In closed-loop mode, the closed-loop artificial pancreas system can provide more accurate blood glucose monitoring and insulin infusion.

In summary, the invention discloses a closed-loop artificial pancreas system for safe infusion, during a long period of real-time blood glucose value loss, the closed-loop artificial pancreas system can enter a learning mode or a safe mode. Based on historical blood glucose values and finger blood glucose values, it continues to calculate the required insulin infusion amount for the patient and infuses an appropriate amount of insulin to the patient. It will not infuse an inappropriate amount of insulin or even interrupt insulin infusion due to the lack of real-time blood glucose value, avoiding life-threatening situations for the patient and benefiting the patient's treatment.

While the invention has been described in detail with reference to the specific embodiments of the invention, it should be understood that it will be appreciated by those skilled in the art that the above embodiments may be modified without departing from the scope and spirit of the invention. The scope of the invention is defined by the appended claims.

**Claims**

1. A closed-loop artificial pancreas system, comprising:

   A detection mechanism for monitoring real-time blood glucose values.
   A program mechanism, used to receive real-time blood glucose values from the detection mechanism and calculate insulin infusion volume based on the real-time blood glucose.
   And an infusion mechanism, used to implement insulin infusion.
   Wherein, when the program mechanism does not receive the real-time blood glucose value for the first period of time, the closed-loop artificial pancreas system switches from the first mode to the second mode for the second period of time. In the second mode, the closed-loop artificial pancreas system calculates the estimated blood glucose value, calculates the estimated insulin infusion volume based on the estimated blood glucose value, and completes the insulin infusion based on the estimated insulin infusion volume.

2. The closed-loop artificial pancreas system of claim 1, wherein,
   the estimated blood glucose value is calculated based on real-time blood glucose values from the last period of time.

3. The closed-loop artificial pancreas system of claim 2, wherein,
   it further comprising providing finger blood glucose values, the estimated blood glucose values being calculated based on finger blood glucose values.

4. The closed-loop artificial pancreas system of claim 3, wherein,
   when estimating blood glucose values based on finger blood glucose values, the second period of time can be extended.

5. The closed-loop artificial pancreas system of claim 3, wherein,
   the closed-loop artificial pancreas system further comprises a preset blood glucose threshold, and different infusion strategies based on the comparison relationship between finger blood glucose values and the blood glucose threshold.

6. The closed-loop artificial pancreas system of claim 5, wherein,
   the infusion strategy includes administering a high dose, reducing insulin infusion, and pausing insulin infusion.

7. The closed-loop artificial pancreas system of claim 3, wherein,
   if meals are taken during the second period, at least one finger blood glucose value before the meal and at least one finger blood glucose value after the meal are provided, and the dietary high-dose before the meal is calculated based on the finger blood glucose value before the meal, and the dietary high-dose after the meal is calculated based on the finger blood glucose value after the meal, respectively.

8. The closed-loop artificial pancreas system of claim 7, wherein,
the finger blood glucose value before the meal is provided 0-20 minutes before the meal, and the finger blood glucose value after the meal is provided 0-150 minutes after the meal.

9. The closed-loop artificial pancreas system of claim 1, wherein,
the calculation of estimated blood glucose value and estimated insulin infusion volume value can be repeated during the second period of time.

10. The closed-loop artificial pancreas system of claim 1, wherein,
if the program mechanism still does not receive the real-time blood glucose value after the second period of time, the closed-loop artificial pancreas system switches to the third mode for the third period of time.

11. The closed-loop artificial pancreas system of claim 10, wherein,
in the third mode, the insulin infusion amount is 0~30% of the estimated insulin infusion volume value.

12. The closed-loop artificial pancreas system of claim 11, wherein,
in the third mode, the insulin infusion amount is 0~10% of the estimated insulin infusion volume value.

13. The closed-loop artificial pancreas system of claim 10, wherein,
the third period of time is from 0 to 48 hours.

14. The closed-loop artificial pancreas system of claim 1, wherein,
the first period of time is 0-24 hours.

15. The closed-loop artificial pancreas system of claim 1, wherein,
the second period of time is from 0 to 48 hours.

16. The closed-loop artificial pancreas system of claim 1-15, wherein,
when the closed-loop artificial pancreas system is in the second mode or the third mode, if the program mechanism recovers to receive the real-time blood glucose value, the closed-loop artificial pancreas system switches to the first mode.

**FIG.1**

**FIG.2**

302

3022

3011

3012

301

3013

3012

**FIG.3**

410

400

420

10

**FIG.4a**

410

400

420

**FIG.4b**

CGM/Insulin Pump ⟷ PDM ⟷ Computer ⟷ Remote server

**FIG.5a**

CGM ⟷ PDM ⟷ Smartphone ⟷ Remote server

**FIG.5b**

CGM ⟷ PDM1

Insulin Pump ⟷ PDM2 ⟷ Computer ⟷ Remote server

**FIG.5c**

CGM ⟷ PDM1

Insulin Pump ⟷ PDM2 ⟷ Smartphone ⟷ Remote server

**FIG.5d**

CGM and /or Insulin Pump ⟷ Smartphone ⟷ Remote server

**FIG.6**

Smartphone ← → Remote server

CGM and /or
Insulin Pump

PDM

**FIG.7**

801 — Remote server sends the update information to the smartphone

802 — Smartphone receives the update information

803 — Smartphone proceeds the updating step

804 — Smartphone reports the update result to the remote server

**FIG.8**

**FIG.9a & FIG.9b**

**FIG.9c & FIG.9d**

**FIG.10**

## INTERNATIONAL SEARCH REPORT

| International application No. |
|---|
| **PCT/CN2024/095389** |

| **A. CLASSIFICATION OF SUBJECT MATTER** |
|---|
| G16H20/17(2018.01)i |
| According to International Patent Classification (IPC) or to both national classification and IPC |

| **B. FIELDS SEARCHED** |
|---|
| Minimum documentation searched (classification system followed by classification symbols) |
| IPC:G16H、A61M、A61B |
| Documentation searched other than minimum documentation to the extent that such documents are included in the fields searched |
| Electronic data base consulted during the international search (name of data base and, where practicable, search terms used) |
| CNABS; CNTXT; VEN; USTXT; WOTXT; EPTXT; CNKI; IEEE: 丢失, 缺失, 信号, 估算, 血糖, 胰岛素, 指尖, 餐; lost, lack of, signal, estimate, blood glucose, insulin, fingertip, meals |

| **C. DOCUMENTS CONSIDERED TO BE RELEVANT** |
|---|

| Category* | Citation of document, with indication, where appropriate, of the relevant passages | Relevant to claim No. |
|---|---|---|
| X | CN 116139367 A (MEDTRUM TECHNOLOGIES INC.) 23 May 2023 (2023-05-23) description, paragraphs 6-489 | 1-16 |
| X | CN 116052822 A (MEDTRUM TECHNOLOGIES INC.) 02 May 2023 (2023-05-02) description, paragraphs 32-484 | 1-16 |
| A | CN 111246898 A (MEDTRONIC MINIMED, INC.) 05 June 2020 (2020-06-05) entire document | 1-16 |
| A | US 2013190583 A1 (MEDTRONIC MINIMED, INC.) 25 July 2013 (2013-07-25) entire document | 1-16 |

☐ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **05 August 2024** | **27 August 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT
### Information on patent family members

| International application No. |
| --- |
| **PCT/CN2024/095389** |

| Patent document cited in search report | | | Publication date (day/month/year) | Patent family member(s) | | | Publication date (day/month/year) |
| --- | --- | --- | --- | --- | --- | --- | --- |
| CN | 116139367 | A | 23 May 2023 | None | | | |
| CN | 116052822 | A | 02 May 2023 | None | | | |
| CN | 111246898 | A | 05 June 2020 | US | 2020101222 | A1 | 02 April 2020 |
| | | | | US | 10894126 | B2 | 19 January 2021 |
| | | | | KR | 20210072758 | A | 17 June 2021 |
| | | | | EP | 3856281 | A1 | 04 August 2021 |
| | | | | US | 2021093783 | A1 | 01 April 2021 |
| | | | | US | 11660394 | B2 | 30 May 2023 |
| | | | | AU | 2019349568 | A1 | 11 March 2021 |
| | | | | JP | 2022502159 | A | 11 January 2022 |
| | | | | US | 2023248910 | A1 | 10 August 2023 |
| | | | | CA | 3110070 | A1 | 02 April 2020 |
| | | | | WO | 2020068188 | A1 | 02 April 2020 |
| | | | | CN | 111246898 | B | 08 April 2022 |
| US | 2013190583 | A1 | 25 July 2013 | US | 2020289037 | A1 | 17 September 2020 |
| | | | | US | 11896370 | B2 | 13 February 2024 |
| | | | | US | 10694983 | B2 | 30 June 2020 |

Form PCT/ISA/210 (patent family annex) (July 2022)